# EUROPEAN PATENT APPLICATION

(11) **EP 4 834 262 A1**
(43) Date of publication of application: **04.11.2026**
(21) Application number: 26175758.7
(22) Date of filing: 30.04.2026
(51) Int. Cl.: A61B 18/14, A61B 18/00

(54) **NEEDLE-TYPE MYOCARDIAL ABLATION CATHETER AND PULSE ABLATION SYSTEM**

(30) Priority: 30.04.2025 CN 202510568413
(71) Applicant: Apt Medical Hunan Inc., Xiangtan, Hunan 411499 (CN); Apt Medical (Shanghai) Inc., Shanghai 201702 (CN)
(72) Inventor: XIE, Wei, Hunan, 410000 (CN); LIANG, Huishan, Shanghai, 200001 (CN); ZHANG, Hanwei, Shanghai, 201108 (CN); LIU, Miao, Hunan, 421600 (CN); ZHOU, Tuo, Shanghai, 200233 (CN)
(74) Representative: Betten & Resch

(57) **Abstract**

The present disclosure provides a needle-type myocardial ablation catheter and a pulse ablation system. The needle-type myocardial ablation catheter comprises a catheter body component, wherein the catheter body component comprises a head end component and a handle component; the head end component comprises an ablation needle and a plurality of electrodes; the plurality of electrodes can form a plurality of discharge modes; the ablation needle is movably disposed in the catheter body component, such that the ablation needle can extend out from a head end of the catheter body component and extend toward a direction away from the catheter body component when ablation is required; and the handle component is provided with a needle extension detection structure therein to acquire a needle extension length of the ablation needle. The embodiments of the present disclosure can achieve reliable positioning of an ablation needle position, and in combination with a magnetic sensor, can realize three-dimensional real-time visualization of a needle extension length.

## Description

### Cross-Reference to Related Application

The present disclosure claims all rights to the Chinese patent application No. 202510568413.8 filed to the National Intellectual Property Administration of the People's Republic of China on April 30, 2025 and entitled "NEEDLE-TYPE MYOCARDIAL ABLATION CATHETER AND PULSE ABLATION SYSTEM", which is incorporated herein by reference in its entirety.

### Technical Field

The present disclosure relates to the technical field of ablation apparatuses, and in particular, to a needle-type myocardial ablation catheter and a pulse ablation system.

### Background

Hypertrophic cardiomyopathy (HCM) is a hereditary cardiomyopathy characterized by asymmetric ventricular hypertrophy. According to whether there is an obstruction in the left ventricular outflow tract, the hypertrophic cardiomyopathy is classified into obstructive hypertrophic cardiomyopathy and non-obstructive hypertrophic cardiomyopathy. The treatment of the HCM primarily aims to alleviate symptoms, reduce complications, and prevent sudden death. The main objective for treating the obstructive hypertrophic cardiomyopathy is to alleviate obstruction-related symptoms, and commonly-used drugs in the past include β-blockers and CCBs. These drugs may partially alleviate obstructions. However, since these drugs do not directly target the lesions caused by the fundamental sarcomere protein mutation, these drugs cannot sufficiently improve the symptoms of patients and may lead to a reduction in the heart rate and blood pressure in patients. Thus, the medication has limited efficacy for patients with severe obstructive hypertrophic cardiomyopathy. For patients with severe symptoms and significant outflow tract pressure difference after adequate medication, surgical operations (septal myectomy) or interventional treatment (e.g., anhydrous ethanol ablation or radiofrequency ablation) needs to be taken into consideration.

Surgical treatment involves directly excising part of the myocardium by surgery to improve local hypertrophy, but is difficult in operation. Thoracotomy, general anesthesia, and extracorporeal circulation are required, with prolonged hospitalization and slow recovery. The surgical treatment is technically demanding, requires specialized expertise, and is less acceptable by most patients. Anhydrous ethanol interventricular septal ablation involves injecting anhydrous ethanol, via a catheter, into the septal branch vessels supplying the septal myocardial tissue to create artificial septal myocardial infarction, so as to alleviate the left ventricular outflow tract obstruction. However, anhydrous ethanol ablation has caused unpredictable complications, mainly including: 1, large-area myocardial infarction leading to heart failure and formation of ventricular aneurysm; 2, large-area necrosis of the ventricular septum leading to cardiac perforation; 3, involvement of the cardiac conduction bundle leading to cardiac arrest or atrioventricular block in patients, thereby requiring implantation of a cardiac pacemaker; and 4, establishment of collateral circulation leading to the thickening of the necrotic myocardial cells again, resulting in disease recurrence.

For most radiofrequency ablation routes, radiofrequency ablation needles or interventional catheters are used, but they have the following defects due to the characteristics of radiofrequency energy: the penetration into fibrotic myocardium is poor; perfusion is required, wherein a small perfusion volume may generate micro-bubbles, while a large perfusion volume may affect an ablation effect; perfusion increases the heart burden on patients with heart failure; and potential damage to the left bundle branch may occur.

### Summary

In order to solve the problems existing in surgical operations (septal myectomy) or interventional treatment for hypertrophic cardiomyopathy in the related art, the present disclosure provides a needle-type myocardial ablation catheter and a pulse ablation system.

In view of this, one aspect of embodiments of the present disclosure provides a needle-type myocardial ablation catheter, comprising a catheter body component, wherein the catheter body component comprises a head end component and a handle component; the head end component comprises an ablation needle and a plurality of electrodes; the plurality of electrodes can form a plurality of discharge modes; the ablation needle is movably disposed in the catheter body component, such that the ablation needle can extend out from a head end of the catheter body component and extend toward a direction away from the catheter body component when ablation is required; and the handle component is provided with a needle extension detection structure therein to acquire a needle extension length of the ablation needle.

In some embodiments, the plurality of electrodes comprise a terminal electrode and at least one ring electrode, and the terminal electrode and the ring electrode are separately paired with the ablation needle to form two electrode pairs.

In some embodiments, the terminal electrode is paired with the ablation needle to form a first discharge mode, and the ring electrode is paired with the ablation needle to form a second discharge mode.

In some embodiments, the ring electrode is disposed on the catheter body component at a predetermined distance from the terminal electrode, and when there are a plurality of ring electrodes, a spacing between two adjacent ones of the plurality of ring electrodes is equal.

In some embodiments, a magnetic sensor is disposed in the head end component; the magnetic sensor is disposed inside the catheter body component; the magnetic sensor is in a long strip shape and is arranged in a length direction of the catheter body component; and the magnetic sensor is configured to cooperate with the terminal electrode and/or the ring electrode to achieve rapid positioning.

In some embodiments, the terminal electrode is disposed on an axial outer side of a head end of the catheter body component, and comprises a first portion and a second portion; the terminal electrode is connected to the catheter body component via the second portion; the second portion is of a threaded knurling structure so as to be integrally formed with the catheter body component by means of heat fusion; the terminal electrode further comprises a through hole; and the through hole axially penetrates through the first portion and the second portion, and is communicated with an internal cavity of the catheter body component, so as to allow the ablation needle to pass through.

In some embodiments, an insulating layer is disposed on a surface of the ablation needle, and covers a middle portion of the ablation needle; an insulating sleeve is further disposed in the through hole; and a tail end of the insulating sleeve is fixed relative to an inner cavity wall of the catheter body component.

In some embodiments, a tail end of the catheter body component is connected to the handle component, and the handle component is further provided with a connector.

In some embodiments, a tail end of the ablation needle is connected to an ablation needle connection portion, and the ablation needle connection portion is connected to the handle component.

In some embodiments, the ablation needle connection portion is provided with a cavity therein; an ablation needle electrode lead is disposed in the cavity; one end of the ablation needle electrode lead is welded to the tail end of the ablation needle, and the other end of the ablation needle electrode lead extends and is welded to the connector.

In some embodiments, the catheter body component comprises an adjustable bending segment and a catheter body segment; a head end of the adjustable bending segment is connected to the head end component; a tail end of the adjustable bending segment is in heat fusion connection with the catheter body segment; a bending adjustment pull ring is disposed inside a junction between the adjustable bending segment and the head end component; and two bending adjustment pull wires are symmetrically arranged on both sides of the bending adjustment pull ring.

In some embodiments, the adjustable bending segment is of a multi-cavity structure that comprises at least four cavities, which are a first cavity, a second cavity, a third cavity, and a fourth cavity respectively; the first cavity and the second cavity are configured to allow the two bending adjustment pull wires to pass through; and the third cavity and the fourth cavity serve as a channel for the ablation needle connection portion to move and a channel for accommodating an electrode lead respectively.

In some embodiments, the handle component comprises a handle housing; a bending adjustment button is disposed at a head end of the handle housing; the handle housing is provided with a sliding button and a needle extension scale; the needle extension scale serves as an identifier to indicate the needle extension length of the ablation needle; and the needle extension detection structure is disposed in the handle housing.

In some embodiments, the bending adjustment button is a push-pull button; the bending adjustment pull wire extends from an internal cavity of the catheter body component to the handle component and is bidirectionally connected to the bending adjustment button; and the adjustable bending segment deflects to a single direction by moving the push-pull button.

In some embodiments, the bending adjustment button is a rotary button, and the adjustable bending segment is controlled by rotating the button to achieve bidirectional deflection.

In some embodiments, the needle extension detection structure comprises a connection sliding block and a sliding rheostat; the sliding button, the connection sliding block, and the sliding rheostat are connected in sequence; a rheostat lead is disposed at a tail portion of the sliding rheostat; the rheostat lead is connected to the connector; by pushing or pulling the sliding button, the ablation needle is able to be driven to extend and withdraw and the sliding rheostat is driven to allow corresponding displacement of a sliding piece inside the sliding rheostat, so as to change a resistance value of an active segment of the sliding rheostat; and a correspondence relationship between the needle extension length of the ablation needle and the resistance value of the sliding rheostat is calibrated.

In some embodiments, the handle component is connected to an perfusion tube; the perfusion tube is configured to supply a saline solution to the head end component; one end of the perfusion tube is connected to an end of a cavity in the adjustable bending segment corresponding to the ablation needle connection portion, extends to a hemostatic valve connector in the handle component, and is led out from a tail end of the handle component; and the connector is configured to exchange data with an external device end via a cable.

Another aspect of the embodiments of the present disclosure provides a pulse ablation system, comprising the needle-type myocardial ablation catheter described in any one of the above embodiments, a pulse ablation instrument, and a three-dimensional mapping system, wherein the pulse ablation instrument is configured to provide energy to the needle-type myocardial ablation catheter.

The embodiments of the present disclosure can achieve reliable positioning of an ablation needle position, and in combination with a magnetic sensor, can realize three-dimensional real-time visualization of a needle extension length.

In order to make the objectives, features, and advantages of the embodiments of the present disclosure more obvious and easier to understand, preferred embodiments are described in detail below with reference to the drawings.

### Brief Description of the Drawings

In the drawings that are not necessarily drawn to scale, the same reference numerals may describe similar components in different views. Different instances of similar components may be indicated by the same reference numerals with letter suffixes or different letter suffixes. The drawings illustrate various embodiments by way of example rather than limitation, and are intended to describe the disclosed embodiments in conjunction with the description and the claims. In appropriate cases, the same reference numerals are used in all drawings to refer to the same or similar parts. Such embodiments are illustrative and are not intended to be exhaustive or exclusive embodiments of the apparatus or method hereinabove. The drawings illustrated herein are used to provide further understanding of the present disclosure and constitute a portion of the present application, and the schematic embodiments of the present disclosure and description thereof are used to explain the present disclosure and do not constitute improper limitation to the present disclosure. In the drawings:
Fig. 1 is a schematic connection diagram of a pulse ablation system according to embodiments of the present disclosure;
Fig. 2 is a schematic structural diagram of a needle-type myocardial ablation catheter according to embodiments of the present disclosure;
Fig. 3 is a schematic structural diagram of a head end component in a needle-type myocardial ablation catheter according to embodiments of the present disclosure;
Fig. 4 is a cross-sectional view of a head end component in a needle-type myocardial ablation catheter according to embodiments of the present disclosure;
Fig. 5 is a schematic structural diagram of an ablation needle and an ablation needle connection portion in a needle-type myocardial ablation catheter according to embodiments of the present disclosure;
Fig. 6 is a schematic structural diagram of a catheter body component in a needle-type myocardial ablation catheter according to embodiments of the present disclosure;
Fig. 7 is a cross-sectional view of an adjustable bending segment of a catheter body component in a needle-type myocardial ablation catheter according to embodiments of the present disclosure;
Fig. 8 is a schematic diagram of unidirectional bending adjustment of a needle-type myocardial ablation catheter according to embodiments of the present disclosure;
Fig. 9 is a schematic structural diagram of a handle component in a needle-type myocardial ablation catheter according to embodiments of the present disclosure;
Fig. 10 is a first schematic diagram of a needle extension detection structure of a handle component in a needle-type myocardial ablation catheter according to embodiments of the present disclosure; and
Fig. 11 is a second schematic diagram of a needle extension detection structure of a handle component in a needle-type myocardial ablation catheter according to embodiments of the present disclosure.

The drawings hereinabove comprise the following reference numerals:
1-Head end component; 11-Ablation needle; 12-Terminal electrode; 121-First portion; 122-Second portion; 13-First ring electrode; 14-Second ring electrode; 15-Third ring electrode; 16-Magnetic sensor; 17-Insulating layer; 18-Insulating sleeve; 19-Ablation needle connection portion; 2-Catheter body component; 21-Adjustable bending segment; 211-Bending adjustment pull ring; 212-Bending adjustment pull wire; 213-First cavity; 214-Second cavity; 215-Third cavity; 216-Fourth cavity; 22-Catheter body segment; 3-Handle component; 31-Bending adjustment button; 32-Sliding button; 33-Needle extension scale; 34-Handle housing; 35-Connection sliding block; 36-Sliding rheostat; 4-Perfusion tube; 5-Connector; 10-Needle-type myocardial ablation catheter; 20-Pulse ablation instrument; and 30-Three-dimensional mapping system.

### Detailed Description of the Embodiments

Specific embodiments of the present disclosure are described below in detail with reference to the drawings, but are not intended to limit the present disclosure.

It should be understood that various modifications may be made to the embodiments disclosed herein. Therefore, the description hereinabove should not be construed as limiting, but merely as an example of an embodiment. A person skilled in the art will be able to conceive of other modifications within the scope and spirit of the present disclosure.

The drawings included in the specification and forming part thereof illustrate embodiments of the present disclosure and, together with the general description of the present disclosure provided above and the detailed description of the embodiments provided below, serve to explain the principles of the present disclosure.

These and other characteristics of the present disclosure will become apparent from the following description of preferred embodiments, which are provided as non-limiting examples with reference to the drawings.

It should also be understood that although the present disclosure has been described with reference to some specific examples, a person skilled in the art can determine many other equivalent forms of the present disclosure that possess the features described in the claims and are therefore all within the scope of protection defined thereby.

When considered in conjunction with the drawings and in view of the following detailed description, the above and other aspects, features, and advantages of the present disclosure will become more apparent.

The specific embodiments of the present disclosure will be described below with reference to the drawings. However, it should be understood that the embodiments disclosed are merely examples of the present disclosure and may be implemented in various ways. Functions and structures that are well known and/or repetitive are not described in detail to avoid unnecessary or redundant details that may obscure the present disclosure. Therefore, the specific structural and functional details disclosed herein are not intended to be limiting, but are provided merely as a basis and a representative basis of the claims for teaching a person skilled in the art in the use of the present disclosure in a variety of substantially arbitrary and suitable detailed structures.

It is to be noted that the terms "first", "second", and the like in the description, claims, and drawings hereinabove of the present disclosure are used to distinguish similar objects, and are not necessarily used to describe a specific order or sequence. It is to be understood that the data used herein may be interchanged under appropriate circumstances, such that the embodiments of the present disclosure described herein can be implemented in sequences other than those illustrated or described. In addition, the terms "comprise" and "have" and any variations thereof are intended to cover non-exclusive inclusion. For example, a process, method, system, product, or device comprising a series of steps or units is not necessarily limited to the steps or units explicitly listed, but may comprise other steps or units not explicitly listed or inherently present in such processes, methods, products, or devices.

The present specification may use phrases such as "in an embodiment", "in another embodiment", "in yet another embodiment", or "in other embodiments", all of which may refer to one or more of the same or different embodiments according to the present disclosure.

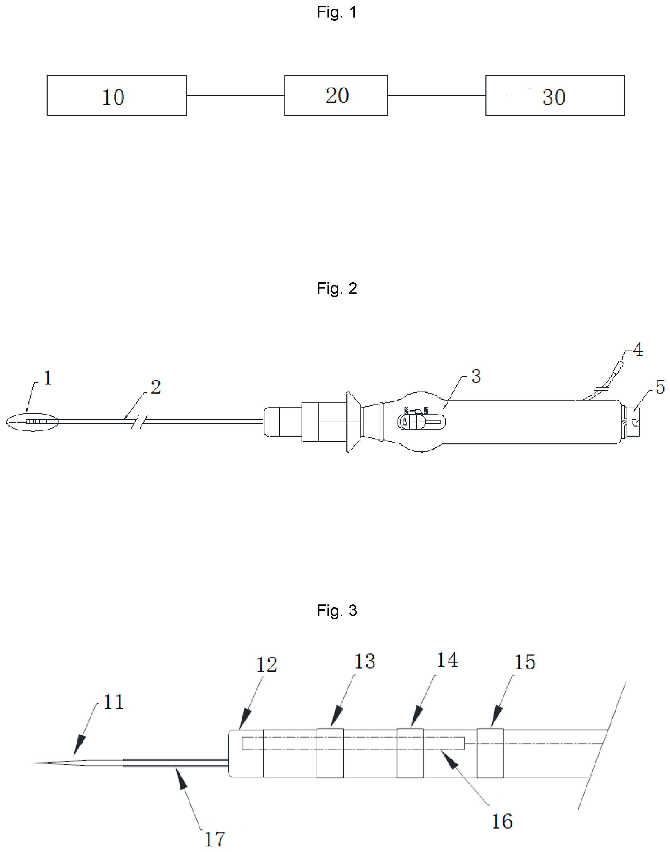

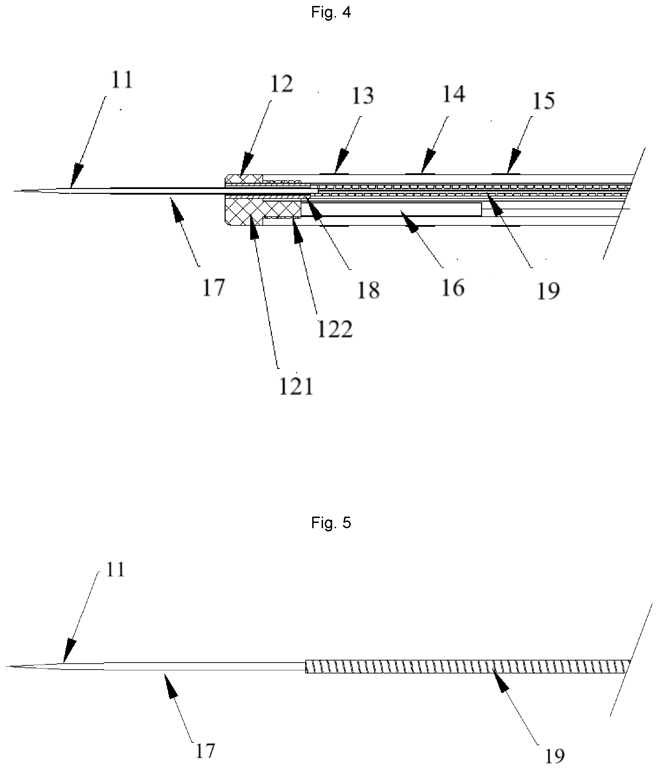

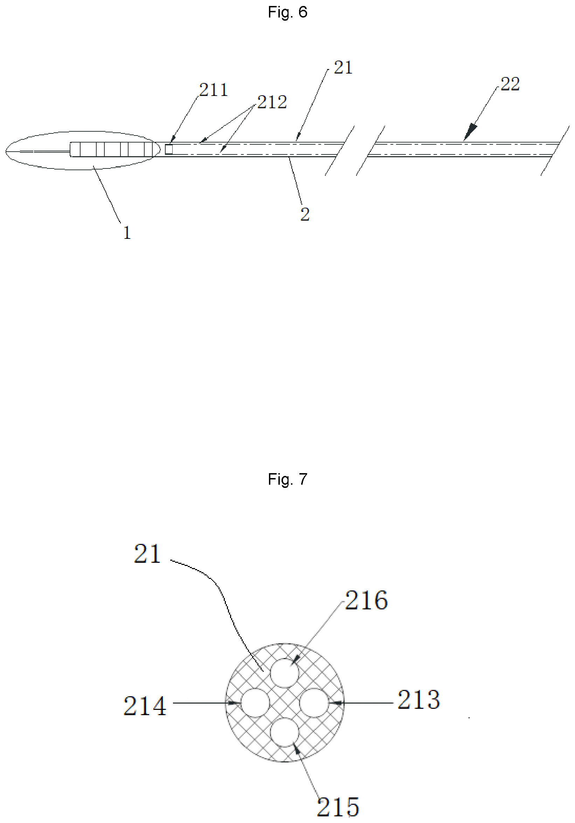

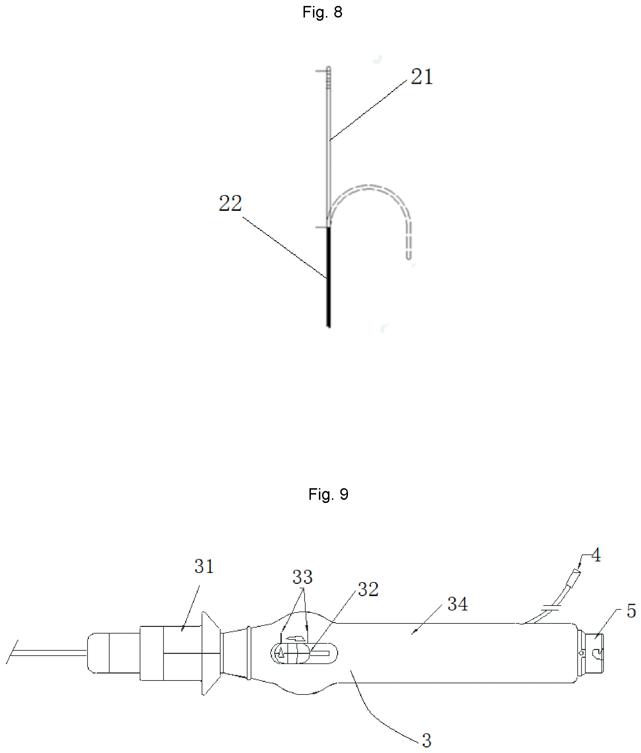

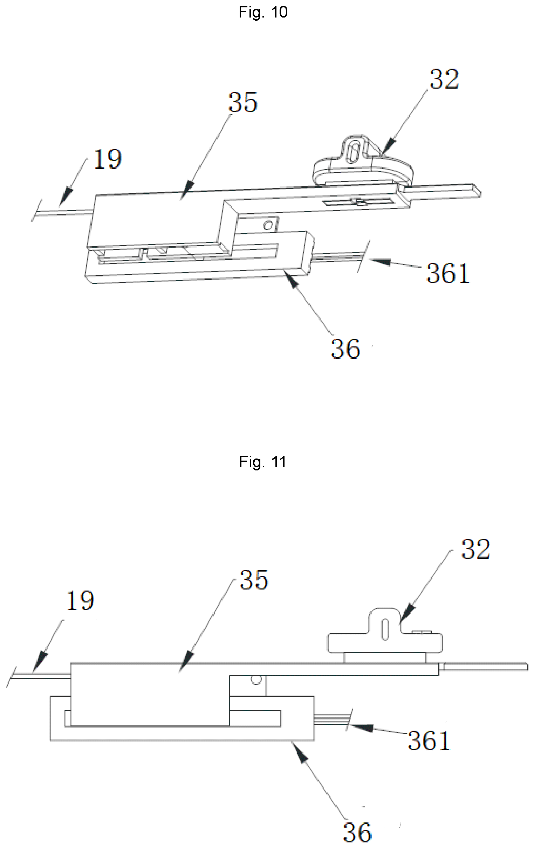

Embodiments of the present disclosure provide a needle-type myocardial ablation catheter, which is configured to achieve ventricular pulse ablation during treatment of hypertrophic cardiomyopathy. The needle-type myocardial ablation catheter is applicable to a pulse ablation system. As shown in Fig. 1 to Fig. 11, the pulse ablation system comprises the needle-type myocardial ablation catheter 10, a pulse ablation instrument 20, and a three-dimensional mapping system 30, wherein the needle-type myocardial ablation catheter 10 is connected to the three-dimensional mapping system 30 via the pulse ablation instrument 20 by means of, for example, cables, and the pulse ablation instrument 20 is configured to provide energy for the needle-type myocardial ablation catheter 10.

As shown in Fig. 2, the needle-type myocardial ablation catheter 10 can be configured to treat a patient with hypertrophic obstructive cardiomyopathy, and perform pulse ablation on partial hypertrophic myocardium in a form of vascular intervention to cause irreversible electroporation in a target myocardial tissue, thereby achieving a volume reduction effect of the hypertrophic myocardium. Pulsed field ablation (PFA) adopted herein releases ablation energy by designing an appropriate pulsed electric field and applying a plurality of short-duration and high-voltage electrical pulses, causing an ablation process to be non-thermal ablation (without Joule heating) to effectively induce electroporation of myocardial cells, so as to allow extracellular cation to enter the cells, thereby causing fragmentation and death of the myocardial cells, such that the pulsed field ablation is therefore also referred to as irreversible electroporation.

Specifically, the needle-type myocardial ablation catheter 10 comprises a catheter body component 2, wherein a head end component 1 is disposed at a head end of the catheter body component 2, a tail end of the catheter body component 2 is connected to a handle component 3, the handle component 3 is connected to a perfusion tube 4, and the handle component 3 is further provided with a connector 5.

Specifically, the head end component 1 is located at a distal end side of the catheter body component 2 and is integrally formed with the head end of the catheter body component 2, for example, by means of heat fusion connection. The tail end of the catheter body component 2 extends into the interior of the handle component 3, and the perfusion tube 4 and the connector 5 may be arranged at a tail end of the handle component 3.

In combination with Fig. 1 and Fig. 2, in the pulse ablation system, pulse energy is provided for the needle-type myocardial ablation catheter 10 by the pulse ablation instrument 20, and a target myocardial tissue is subjected to pulse ablation by using the head end component 1 located at the head end of the catheter body component 2, so as to achieve a therapeutic purpose.

Furthermore, a state and position of the needle-type myocardial ablation catheter 10 in a human body may be monitored in real time by using the matching three-dimensional mapping system 30, so as to guide an operator to quickly locate an ablation site, such that completion of surgery is facilitated by accurately positioning an ablation position.

Further, the head end component 1 comprises an ablation needle 11 configured to achieve pulse ablation, a plurality of electrodes, and a magnetic sensor 16, wherein the ablation needle 11 is movably disposed in the catheter body component 2, can extend from the head end of the catheter body component 2, and extends toward a direction away from the catheter body component 2 when ablation is required. The ablation needle 11 here can extend different lengths relative to the catheter body component 2 according to the ablation site, facilitating an operation of the operator. In this embodiment, ablation is performed in a single-needle manner. The single-needle design is less in mechanical damage to a target tissue compared with helical structures and dual-needle structures in the related art, thereby reducing surgical risks.

Specifically, the ablation needle 11 is made of a nickel-titanium material, with a diameter φ of 0.3-0.5 mm and a total length of 20-30 mm. The ablation needle 11 in this embodiment may be obtained by grinding a nickel-titanium wire to form a tip. The ablation needle 11 here made of the nickel-titanium material has higher bend-resistant performance and toughness, thereby ensuring the safety of the surgery.

Further, in order to enhance the development performance of the ablation needle in the surgery, a surface of the ablation needle 11 is plated with gold, a gold-plated development layer is coated on the surface of the ablation needle 11, and a thickness of the gold-plated development layer is 0.1-50 µm. Herein, disposing the gold-plated development layer on the ablation needle 11 can replace a manner of sleeving an ultra-thin platinum-iridium tube outside an ablation needle, such that the development performance of the ablation needle can be ensured on the basis of structural simplification.

Furthermore, an insulating layer 17 is further disposed on the surface of the ablation needle 11, and a material of the insulating layer 17 is parylene, for example, deposited on the surface of the ablation needle 11 by means of vacuum meteorological deposition. Certainly, the material of the insulating layer 17 may not be limited to the parylene coating, and may also be replaced with a PTFE coating or materials such as PTFE, PI, polyurethane, polyether-block polyamide, etc. thereby achieving an insulation purpose.

The insulating layer 17 here particularly covers a middle portion of the ablation needle 11. For example, the covered portion is a portion between a first position 2-10 mm from a needle tip of the ablation needle 11 and a second position 2-5 mm from a needle tail of the ablation needle 11. In this embodiment, the thickness and length of the insulating layer 17 may be set according to actual requirements. Preferably, the thickness of the insulating layer 17 is 20-50 µm.

Further, the electrode comprises a terminal electrode 12 and at least one ring electrode, wherein the terminal electrode 12 here is disposed on an axial outer side of the head end of the catheter body component 2, the terminal electrode 12 is made of a platinum-iridium material, and the terminal electrode 12 may be used as a discharge electrode or a mapping electrode during ablation surgery. One or more ring electrodes here may be provided.

A length of the terminal electrode 12 exposed to the outside is preferably 1.5 mm-2 mm. Furthermore, a tail end of the terminal electrode 12 is connected to a terminal electrode lead wire, and the terminal electrode lead wire extends to the tail end of the handle component 3 via an internal cavity of the catheter body component 2 and is connected to the connector 5.

The terminal electrode 12 and the ring electrode in this embodiment may respectively paired with the ablation needle 11. When serving as two pairs of electrode pairs, the terminal electrode and the ring electrode may be not only configured to discharge to achieve ablation, but also configured to achieve mapping, etc. The arrangement of using the terminal electrode 12 in cooperation with the plurality of the ring electrodes can also provide a plurality of discharge modes, thereby providing more options for the operator. This embodiment can meet requirements such as discharge ablation, etc. by adopting the structure of the plurality of electrodes, and can also meet requirements such as mapping, etc.

Specifically, this embodiment may have two discharge modes, which are a first discharge mode and a second discharge mode. In the first discharge mode, the ablation needle 11 and the terminal electrode 12 discharge at the same time; an ablation lesion generated in this mode may start from an endocardial surface at a puncture site and extends into a deep myocardial tissue; and due to a short distance between the ablation needle 11 and the terminal electrode 12, using a high energy output is liable to generate an electric arc, resulting in thermal damage to the myocardial tissue. In the second discharge mode, the ablation needle 11 and the ring electrode discharge at the same time; the ablation lesion generated in this mode starts at a position approximately 1 mm from the endocardial surface at the puncture site, forms an ellipsoid using the ablation needle 11 as an axis, and extends into the deep myocardial tissue; and due to the long distance between the ablation needle 11 and the ring electrode, and compared with the first discharge mode, using the high energy output is less liable to generate the electric arc, causing less damage to the endocardial surface at a puncture side. For example, when the ablation needle is inserted into a ventricular septum from a right ventricular side for ablation, the discharge mode 2 may protect structures such as the His bundle, etc. on the endocardial surface more effectively, and has higher safety. In practice, the first discharge mode or the second discharge mode may be selected according to ablation requirements during surgery. For example, the first discharge mode is selected when the surface of a needle puncture needs to be damaged, and the second discharge mode 2 is selected when the endocardial surface of the needle puncture needs to be protected.

Further, the terminal electrode 12 comprises a first portion 121 and a second portion 122, wherein the terminal electrode 12 is connected to the catheter body component 2 via the second portion 122, and a radial dimension of the first portion 121 is greater than a radial dimension of the second portion 122. The radial dimension of the first portion 121 is preferably the same as the radial dimension of the catheter body component 2. The first portion 121 and the second portion 122 here are both preferably cylindrical so as to maintain surface consistency with the catheter body component 2. Herein, the structure of the terminal electrode 12 hereinabove may be formed, for example, by means of machining.

Further, the second portion 122 of the terminal electrode 12 here is of a threaded knurling structure so as to be integrally formed with the catheter body component 2 by means of heat fusion, thereby improving the firmness of the head end head end component 1. A threaded knurling tail end of the terminal electrode 12 is firmer and less likely to fall off. Furthermore, the threaded knurling structure adopted by the second portion 122 of the terminal electrode 12 may be replaced with a high-strength pull wire (e.g., made of an SUS304/SUS316 material) welded at the tail end and thermally fused or adhered to the interior of the catheter body component 2, so as to fix the terminal electrode 12 to prevent the terminal electrode from falling off.

The ablation needle 11 is disposed in an internal cavity of the catheter body component 2 and can move to extend through the terminal electrode 12. In order to control movement of the ablation needle 11, a tail end of the ablation needle 11 is connected to an ablation needle connection portion 19, the ablation needle connection portion 19 is connected to the handle component 3, and the ablation needle 11 is controlled to move by operating the ablation needle connection portion 19. Specifically, a tail end of the ablation needle connection portion 19 extends to an interior of the handle component 3 via the internal cavity of the catheter body component 2 and is connected to an operating mechanism on the handle component 3, and the ablation needle 11 is controlled to extend from or withdraw into an end face of the terminal electrode 12 by operating the handle component 3. Preferably, the ablation needle 11 and the ablation needle connection portion 19 here may move freely in the internal cavity of the catheter body component 2.

Further, the ablation needle connection portion 19 is provided with a cavity; an ablation needle electrode lead is disposed in the cavity; one end of the ablation needle electrode lead is welded to the tail end of the ablation needle 11, and the other end of the ablation needle electrode lead extends and is welded to the connector 5. Compared with an integrated needle formed integrally, which is large in internal resistance and poor in pulse ablation effect, this embodiment forms a structure via the ablation needle 11 and the ablation needle connection portion 19 and directly connects a lead to the tail end of the ablation needle, such that the internal resistance of the needle may be effectively reduced, thereby achieving a desired ablation effect.

The ablation needle connection portion 19 here is preferably a thread-cut stainless steel tube, and the tail end of the ablation needle 11 may be welded to the thread-cut stainless steel tube.

Preferably, the thread-cut stainless steel tube here may be replaced with a tightly-wound spring formed by a plurality of strands of stainless steel wire. The tightly-wound spring has good weldability, bendability and pushing performance, and thus also meets various performance requirements. Specifically, compared to the thread-cut stainless steel tube, the tightly-wound spring has the advantage of being softer, and, while ensuring the pushing performance, may also adapt to a catheter bending adjustment requirement. Furthermore, a cut end face of the thread-cut stainless steel tube is prone to deformation, but the tightly-wound spring does not have these defects hereinabove. The tightly-wound spring is pushed in a tube cavity, and there is no gap between spring wires, such that the needle can be smoothly pushed out of a sheath, thereby achieving good pushing performance.

In order to achieve the mounting of the terminal electrode 12 and facilitate movement of the ablation needle 11, the terminal electrode 1 further comprises a through hole, the through hole axially penetrates through the first portion 121 and the second portion 122 and communicate with the internal cavity of the catheter body component 2, and the through hole facilitates free passing through of the ablation needle 11 and protrusion of the ablation needle from an end surface of the terminal electrode 12. A diameter of a channel formed by the through hole here is greater than a diameter of the ablation needle 11, such that a gap between the channel and the ablation needle 11 may allow a saline solution to pass through.

Furthermore, an insulating sleeve 18 is further disposed in the through hole, the insulating sleeve 18 is made of a PI material, and the insulating sleeve 18 here may also be made of materials such as PEEK, TPU, etc. The insulating sleeve 18 is attached to an inner wall of the through hole, and the ablation needle 11 here may pass through the insulating sleeve 18. Furthermore, a tail end of the insulating sleeve 18 is fixed to an inner cavity wall of the catheter body component 2 by means of an adhesive, so as to improve insulation performance inside the terminal electrode 12.

During a pulse ablation operation, a non-insulated portion (a position not covered by the insulating sleeve 18) of the ablation needle 11 near a distal end of the needle tip may serve as a discharge electrode to cooperate with the terminal electrode 12, the ring electrode, etc. to achieve discharge, and a non-insulated portion (a position not covered by the insulating sleeve 18) of the ablation needle 11 near a proximal end of the needle facilitates welding to the electrode lead and the ablation needle connection portion 19.

A needle tail of the ablation needle 11 here is connected to an ablation needle lead, and the ablation needle lead extends from a tube cavity inside the ablation needle connection portion 19 to an interior of the handle component 3 and is connected to the connector 5 at the tail end of the handle component 3. The ablation needle lead is directly welded at the needle tail, causing internal resistance from the ablation needle to the connector end to be small, thereby meeting a low internal resistance requirement for pulse ablation.

In this embodiment, the arrangement of the insulating sleeve 18 and the insulating layer 17 can doubly ensure an insulation withstand voltage effect of the ablation needle 11, and an ablation voltage parameter may be increased during ablation, so as to generate a larger damage range and a greater damage depth.

Further, the catheter body component 2 is provided with at least one ring electrode. The ring electrode can cooperate with the terminal electrode 12 and the ablation needle 11 to achieve discharge ablation and mapping. The ring electrode is disposed on the catheter body component 2 at a predetermined distance from the terminal electrode 12. The ring electrode may be an electrode ring made of a platinum-iridium material, which is sleeved on an outer side of the catheter body component 2, and fixed to the catheter body component 2, for example, by means of glue application or heat fusion. Further, the terminal electrode 12 and the ring electrode may have an axial length of 1-3 mm, preferably 2 mm.

Further, each of the ring electrodes is connected to a ring electrode lead, and the ring electrode lead extends to a tail end of the handle component 3 via the internal cavity of the catheter body component 2 and is connected to the connector 5.

In this embodiment, the number of the ring electrodes may be 1-5. For example, in this embodiment, a first ring electrode 13, a second ring electrode 14, and a third ring electrode 15 may be sequentially arranged on the catheter body component 2 from far to near. Furthermore, when there are a plurality of ring electrodes, a spacing between two adjacent ring electrodes is equal, and the electrode spacing may be 1-5 mm, preferably 2 mm. In this way, the dimension and position of each electrode on the catheter body component 2 may be predetermined, so as to facilitate rapid positioning using the position of the electrode.

Further, the magnetic sensor 16 is disposed inside the catheter body component 2, and for example, is attached to the inner wall of the catheter body component 2 by means of an adhesive. The magnetic sensor 16 is, for example, long-strip-shaped and is arranged in a length direction of the catheter body component 2. A tail end of the magnetic sensor 16 is connected to a magnetic sensor lead, and the magnetic sensor lead extends to the tail end of the handle component 3 via the internal cavity of the catheter body component and is connected to the connector 5.

The magnetic sensor 16 can cause the head end of the catheter body component 2 to achieve positioning during surgery by means of magnetic positioning, and may also display in real time the position of the head end of the needle-type myocardial ablation catheter 10 in the matching three-dimensional mapping system 30, thereby providing reliable guidance for the operator. Specifically, a positional relationship between the magnetic sensor 16 and each of the electrodes is predetermined, such that rapid positioning can be achieved via cooperation with each of the electrodes, that is, by pre-mapping and recording a relative position between the magnetic sensor 16 and each of the electrodes, the real-time position of the head end of the needle-type myocardial ablation catheter 10 during surgery is displayed by the three-dimensional mapping system 30.

Further, the catheter body component 2 comprises an adjustable bending segment 21 and a catheter body segment 22, which are connected in sequence. A head end of the adjustable bending segment 21 here is connected to the head end component 1, and a tail end of the adjustable bending segment 21 is in heat fusion connection with the catheter body segment 22. An outer diameter of the catheter body component 2 here is 8 Fr (1 Fr=1/3 mm). According to a bending pattern of the catheter, a length of the adjustable bending segment 21 in this embodiment is 20-70 mm.

A bending adjustment pull ring 211 is disposed inside a junction between the adjustable bending segment 21 and the head end component 1; two bending adjustment pull wires 212 are welded to both sides of the bending adjustment pull ring 211; and the two bending adjustment pull wires 212 are symmetrically distributed at 180° to each other. The bending adjustment pull ring 211 here is made of an SUS304 material, with a radial dimension being less than the outer diameter of the catheter body component 2. Furthermore, the material of the bending adjustment pull ring 211 may also be replaced with platinum-iridium alloy, SUS316, etc. The bending adjustment pull wire 212 is preferably a stainless steel rope, and may certainly also replaced with stainless steel round wire, stainless steel flat wire, etc. By pulling the bending adjustment pull wire 212, the adjustable bending segment 21 can be driven to bend by the bending adjustment pull ring 211.

The adjustable bending segment 21 here is of a multi-cavity structure to facilitate arrangement of different functional channels such as a channel for the bending adjustment pull wire 212, a channel for the ablation needle 11, a channel for the electrode lead, etc. Specifically, the adjustable bending segment, for example, comprises at least four cavities, which respectively are a first cavity 213, a second cavity 214, a third cavity 215, and a fourth cavity 216, wherein the first cavity 213 and the second cavity 214 are bending adjustment pull wire cavities to allow two of the bending adjustment pull wires 212 to pass through and extend into the interior of the handle component 3; and the third cavity 215 and the fourth cavity 216 are respectively a channel for movement of the ablation needle connection portion 19 of the ablation needle 11 and a channel for accommodating the electrode lead. Certainly, functions of different cavities are exchangeable herein, and no specific limitation is made.

Further, in this embodiment, the handle component 3 is provided with a bending adjustment mechanism and a needle extension detection mechanism. The bending adjustment mechanism here is configured to deflect and bend the head end component 1 up to 180°; and the needle extension detection mechanism is configured to control the extension and withdrawal of the ablation needle 11 and determine a needle extension length of the ablation needle 11 by means of direct vision and simulation.

Specifically, the handle component 3 comprises a handle housing 34, wherein a bending adjustment button 31 is disposed at a head end of the handle housing 34, the handle housing 34 is provided with a sliding button 32, and a needle extension scale 33 is provided on a surface of the handle housing 34 and in a position adjacent to the sliding button 32. The needle extension scale 33 here is formed by silk printing or laser engraving to serve as an identifier to indicate the needle extension length of the ablation needle **11,** thereby providing visual reference for the operator.

The bending adjustment button 31 here is a push-pull button, and bending adjustment of the adjustable bending segment 21 is achieved by moving the push-pull button. Specifically, the bending adjustment pull wire 212 in the adjustable bending segment 21 extends to the handle component 3 via the internal cavity of the catheter body component 2 and is bidirectionally connected to the bending adjustment button 31. The adjustable bending segment 21 may be deflected in a single direction by pushing or pulling the bending adjustment button 31, and a maximum deflection angle here is 180°.

In some embodiments, the push-pull button may certainly be replaced with a rotary button. The bending adjustment of the adjustable bending segment 21 is controlled by rotating the button. The bending pattern of the adjustable bending segment 21 may also be replaced from unidirectional deflection to bidirectional deflection, and the deflection angles are bilaterally symmetrical, with each reaching 180°.

Further, the needle extension detection structure comprises a connection sliding block 35 and a sliding rheostat 36, wherein the sliding button 32, the connection sliding block 35, and the sliding rheostat 36 are sequentially connected. A rheostat lead 361 is disposed at a tail portion of the sliding rheostat 36, and the rheostat lead 361 is connected to the connector 5 by, for example, welding.

The principle of the sliding rheostat 36 here is to change a resistance value by changing a length of a resistance wire. The sliding rheostat generally consists of the resistance wire, a sliding piece, a terminal, a metal rod, a porcelain cylinder, etc., wherein the resistance wire is wound around the insulating porcelain cylinder and externally coated with insulating paint. A length of the resistance wire connected to a circuit may be changed by moving a position of the sliding piece, so as to change the resistance value, thereby achieving a purpose of controlling a current magnitude in the circuit.

In this embodiment, the tail end of the ablation needle connection portion 19 extends into the interior of the handle housing 34 and is fixedly connected to the connection sliding block 35. In this way, the connection sliding block 35 and the ablation needle connection portion 19 can be sequentially driven to move by pushing or pulling the sliding button 32, so as to drive the ablation needle 11 at the head end of the catheter body component 2 to extend or withdraw relative to the end face of the terminal electrode 12, thereby achieving control of the position of the ablation needle 11 during surgery.

Further, while the sliding button 32 is pushed or pulled, the sliding rheostat 36 is also driven to allow corresponding displacement of the sliding piece inside the sliding rheostat, thereby changing a resistance value of an active segment of the sliding rheostat 36. Herein, a correspondence relationship between the needle extension length of the ablation needle 11 and the resistance value of the sliding rheostat 36 may be calibrated, and the relationship between the resistance value and the needle extension length may be quantified and recorded on a controller of a related device end (e.g., the three-dimensional mapping system 30). In this way, during use, data exchange is performed with the device end via a cable, so as to acquire an electrical signal converted from a resistance change caused by pushing or pulling the sliding button 32, such that monitoring functions such as needle extension indication, the needle extension length, etc. are provided via the matching three-dimensional mapping system 30, thereby facilitating the operation of the operator.

The handle component 3 in this embodiment has a simple needle extension structure that meets operational requirements of the operator. The needle extension scale, the needle extension detection structure, etc. provided on the handle component respectively provide, to the operator, reliable positioning between the ablation needle 11 and each of the electrodes from both the catheter end and the device end, and real-time three-dimensional visualization of the needle extension length is realized in combination with the arrangement of the magnetic sensor 16.

Further, the perfusion tube 4 is configured to supply a saline solution to the head end component 1; and one end of the perfusion tube is connected to an end of a cavity in the adjustable bending segment 21 corresponding to the ablation needle connection portion 19, extends to a hemostatic valve connector in the handle component 3, and then is led out from the tail end of the handle component 3, so as to achieve saline solution perfusion of an ablation needle channel before or during surgery.

The connector 5 serves as a data/energy exchange interface. Electrical apparatuses in the needle-type myocardial ablation catheter 10, such as the ablation needle 11, the terminal electrode 12, the ring electrode, the magnetic sensor 16, the sliding rheostat 36, etc., are each connected via corresponding leads that pass through the handle component 3 and are connected to the connector 5, thereby achieving data exchange with the device end such as the three-dimensional mapping system 30 via cables. The connector 5 here may be disposed outside the handle component 3 and connected to a relevant cable, facilitating connection and replacement.

When this embodiment is adopted, considering that a vascular intervention catheter causes less surgical damage to patients compared to treatment catheters used via other access routes, the selection of pulsed ablation energy, due to its characteristics such as tissue selectivity, non-thermal ablation, etc., shows superior safety and effectiveness over a radiofrequency ablation solution. Furthermore, needle-type ablation overcomes a pain point of an insufficient ablation depth faced by other large-head morphology catheters during HCM ablation.

This embodiment serves as the needle-type pulse ablation catheter for vascular intervention, its head end is provided with the ablation needle, the terminal electrode, and the plurality of ring electrodes, and the magnetic sensor is disposed inside the head end. The ablation needle may freely extend and withdraw relative to a channel in the catheter. The ablation needle here is made of the nitinol-titanium material and provided with the insulating layer on its surface, and the non-insulated portion may serve as a discharge electrode and cooperate with the terminal electrode or the ring electrode as an electrode pair to discharge, such that the target myocardial tissue is ablated. A handle end is provided with a needle extension structure and the needle extension detection structure, so as to control needle extension of the ablation needle, and real-time three-dimensional visualization of the needle extension length is achieved by applying the sliding rheostat.

Further, the knurling structure at the tail end of the terminal electrode improves the arrangement firmness of the terminal electrode, and an internal opening provides a suitable channel for the ablation needle, thereby meeting a needle extension requirement. Furthermore, the insulating layer is disposed on the ablation needle, the insulating sleeve is disposed at the opening of the terminal electrode, the arrangement of the insulating sleeve and the insulating layer doubly ensures an insulation withstand voltage effect of the ablation needle, and an ablation voltage parameter may be increased during ablation, so as to generate a larger damage range and a greater damage depth. The head end adopts an adjustable bending structure, which can control bending adjustment of the catheter head end via the handle component, so as to meet requirements of the operator for ablating target tissues at different positions. The needle extension structure of the handle component meets the operation requirements of the operator, and the needle extension scale, the needle extension detection structure, etc. respectively provide, to the operator, reliable positioning between the ablation needle and each of the electrodes from both the catheter end and the three-dimensional device end, and real-time three-dimensional visualization of the needle extension length is realized in combination with the arrangement of the magnetic sensor.

A second embodiment of the present disclosure provides a pulse ablation system. The pulse ablation system comprises a needle-type myocardial ablation catheter 10, a pulse ablation instrument 20, and a three-dimensional mapping system 30, wherein the needle-type myocardial ablation catheter 10 is connected to the three-dimensional mapping system 30 via the pulse ablation instrument 20 by means of, for example, cables, and the pulse ablation instrument 20 is configured to provide energy for the needle-type myocardial ablation catheter 10.

The embodiments of the present disclosure can achieve reliable positioning of an ablation needle position, and in combination with a magnetic sensor, can realize three-dimensional real-time visualization of a needle extension length.

In the embodiments of the present disclosure described above, each embodiment is described with a different focus. Parts that are not elaborated upon in one embodiment can be referred to in the relevant descriptions of other embodiments.

For ease of description, spatially relative terms such as "over", "above", "on upper surface", "upper", and the like may be used herein to describe the spatial positional relationship between one device or feature and another device or feature as illustrated in the drawings. **It** is to be understood that spatially relative terms are intended to include different orientations of the device during use or operation in addition to the orientation depicted in the drawings. For example, if a device in the drawings is inverted, a device described as being "above" or "over" another device or structure is then positioned as "below" or "under" another device or structure. Accordingly, the exemplary term "above" may comprise two orientations "above" and "below". The device can also be positioned in other different ways (rotated 90 degrees or in other orientations), with respective explanations provided for the spatial relative descriptions used here.

In addition to the hereinabove, it is also to be noted that "one embodiment", "another embodiment", "embodiment", etc. used in this specification refer to specific features, structures, or characteristics described in conjunction with the embodiment, which are included in at least one of the embodiments described in the general description of the present application. The same expression appearing in a plurality of places in the specification does not necessarily refer to the same embodiment.

Further, when a specific feature, structure, or characteristic is described in conjunction with any one embodiment, it is intended that such feature, structure, or characteristic implemented in conjunction with other embodiments also falls within the scope of the present disclosure.

In the embodiments hereinabove, each embodiment is described with a different focus. Parts that are not elaborated upon in one embodiment can be referred to in the relevant descriptions of other embodiments.

The above are just preferred embodiments of the present disclosure and are not used for limiting the present disclosure. For a person skilled in the art, the present disclosure may have various modifications and variations. Any modification, equivalent replacement, improvement, and the like made within the spirit and principle of the present disclosure shall be comprised in the scope of protection of the present disclosure.

## Claims

1. A needle-type myocardial ablation catheter, comprising a catheter body component, wherein the catheter body component comprises a head end component and a handle component; the head end component comprises an ablation needle and a plurality of electrodes; the plurality of electrodes are able to form a plurality of discharge modes; the ablation needle is movably disposed in the catheter body component such that the ablation needle is able to extend out from a head end of the catheter body component and extend toward a direction away from the catheter body component when ablation is required; and the handle component is provided with a needle extension detection structure therein to acquire a needle extension length of the ablation needle.

2. The needle-type myocardial ablation catheter according to claim 1, wherein the plurality of electrodes comprise a terminal electrode and at least one ring electrode, and the terminal electrode and the ring electrode are separately paired with the ablation needle to form two electrode pairs, wherein the terminal electrode is paired with the ablation needle to form a first discharge mode, and the ring electrode is paired with the ablation needle to form a second discharge mode.

3. The needle-type myocardial ablation catheter according to claim 2, wherein the ring electrode is disposed on the catheter body component at a predetermined distance from the terminal electrode, and when there are a plurality of ring electrodes, a spacing between two adjacent ones of the plurality of ring electrodes is equal.

4. The needle-type myocardial ablation catheter according to claim 2, wherein a magnetic sensor is disposed in the head end component; the magnetic sensor is disposed inside the catheter body component; the magnetic sensor is in a long strip shape and is arranged in a length direction of the catheter body component; and the magnetic sensor is configured to cooperate with the terminal electrode and/or the ring electrode to achieve rapid positioning.

5. The needle-type myocardial ablation catheter according to claim 2, wherein the terminal electrode is disposed on an axial outer side of a head end of the catheter body component, and comprises a first portion and a second portion; the terminal electrode is connected to the catheter body component via the second portion; the second portion is of a threaded knurling structure so as to be integrally formed with the catheter body component by means of heat fusion; the terminal electrode further comprises a through hole; and the through hole axially penetrates through the first portion and the second portion, and is communicated with an internal cavity of the catheter body component, so as to allow the ablation needle to pass through.

6. The needle-type myocardial ablation catheter according to claim 5, wherein an insulating layer is disposed on a surface of the ablation needle, and covers a middle portion of the ablation needle; an insulating sleeve is further disposed in the through hole; and a tail end of the insulating sleeve is fixed relative to an inner cavity wall of the catheter body component.

7. The needle-type myocardial ablation catheter according to claim 2, wherein a tail end of the catheter body component is connected to the handle component, and the handle component is further provided with a connector, wherein a tail end of the ablation needle is connected to an ablation needle connection portion, and the ablation needle connection portion is connected to the handle component, wherein the ablation needle connection portion is provided with a cavity therein; an ablation needle electrode lead is disposed in the cavity; one end of the ablation needle electrode lead is welded to the tail end of the ablation needle, and the other end of the ablation needle electrode lead extends and is welded to the connector.

8. The needle-type myocardial ablation catheter according to claim 1, wherein the catheter body component comprises an adjustable bending segment and a catheter body segment; a head end of the adjustable bending segment is connected to the head end component; a tail end of the adjustable bending segment is in heat fusion connection with the catheter body segment; a bending adjustment pull ring is disposed inside a junction between the adjustable bending segment and the head end component; and two bending adjustment pull wires are symmetrically arranged on both sides of the bending adjustment pull ring.

9. The needle-type myocardial ablation catheter according to claim 8, wherein the adjustable bending segment is of a multi-cavity structure that comprises at least four cavities, which are a first cavity, a second cavity, a third cavity, and a fourth cavity respectively; the first cavity and the second cavity are configured to allow the two bending adjustment pull wires to pass through; and the third cavity and the fourth cavity serve as a channel for the ablation needle connection portion to move and a channel for accommodating an electrode lead, respectively.

10. The needle-type myocardial ablation catheter according to claim 8, wherein the handle component comprises a handle housing; a bending adjustment button is disposed at a head end of the handle housing; the handle housing is provided with a sliding button and a needle extension scale; the needle extension scale serves as an identifier to indicate the needle extension length of the ablation needle; and the needle extension detection structure is disposed in the handle housing.

11. The needle-type myocardial ablation catheter according to claim 10, wherein the bending adjustment button is a push-pull button; the bending adjustment pull wire extends from an internal cavity of the catheter body component to the handle component and is bidirectionally connected to the bending adjustment button; and the adjustable bending segment deflects to a single direction by moving the push-pull button.

12. The needle-type myocardial ablation catheter according to claim 10, wherein the bending adjustment button is a rotary button, and the adjustable bending segment is controlled by rotating the button to achieve bidirectional deflection.

13. The needle-type myocardial ablation catheter according to claim 10, wherein the needle extension detection structure comprises a connection sliding block and a sliding rheostat; the sliding button, the connection sliding block, and the sliding rheostat are connected in sequence; a rheostat lead is disposed at a tail portion of the sliding rheostat; the rheostat lead is connected to the connector; by pushing or pulling the sliding button, the ablation needle is able to be driven to extend and withdraw and the sliding rheostat is driven to allow corresponding displacement of a sliding piece inside the sliding rheostat, so as to change a resistance value of an active segment of the sliding rheostat; and a correspondence relationship between the needle extension length of the ablation needle and the resistance value of the sliding rheostat is calibrated.

14. The needle-type myocardial ablation catheter according to claim 8, wherein the handle component is connected to an perfusion tube; the perfusion tube is configured to supply a saline solution to the head end component; one end of the perfusion tube is connected to an end of a cavity in the adjustable bending segment corresponding to the ablation needle connection portion, extends to a hemostatic valve connector in the handle component, and is led out from a tail end of the handle component; and the connector is configured to exchange data with an external device end via a cable.

15. A pulse ablation system, comprising the needle-type myocardial ablation catheter according to any one of claims 1 to 14, a pulse ablation instrument, and a three-dimensional mapping system, wherein the pulse ablation instrument is configured to provide energy to the needle-type myocardial ablation catheter.
